(19) Europäisches Patentamt / European Patent Office / Office européen des brevets

(11) **EP 2 198 910 A1**

(12) **EUROPEAN PATENT APPLICATION**

(43) Date of publication:
**23.06.2010 Bulletin 2010/25**

(51) Int Cl.:
*A61M 21/00* (2006.01)      *A47C 21/00* (2006.01)
*A61G 7/00* (2006.01)      *A61H 1/00* (2006.01)

(21) Application number: **08172261.3**

(22) Date of filing: **19.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Koninklijke Philips Electronics N.V.
5621 BA Eindhoven (NL)**

(72) Inventor: **The designation of the inventor has not yet been filed**

(74) Representative: **Damen, Daniel Martijn
Philips
Intellectual Property & Standards
P.O. Box 220
5600 AE Eindhoven (NL)**

(54) **Moving a sleeping surface**

(57)    A system for moving a sleeping surface (10) for a human being (20), comprises at least one actuator (30) for moving the sleeping surface and a movement controller (40) for controlling the actuator. The system further comprises a physiological state estimation unit (50) for estimating a physiological state of the human being on the basis of a sensed value of at least one sensed physiological parameter of said human being, wherein the operation of the movement controller is dependent on the estimated physiological state.

Figure 1

EP 2 198 910 A1

## Description

FIELD OF THE INVENTION

The present invention relates to a system for moving a sleeping surface.

The present invention further relates to a sleeping surface provided with such a system.

The present invention further relates to a method of moving a sleeping surface.

BACKGROUND OF THE INVENTION

Related Art

Sleeplessness is pervasive and in all its forms almost affects the life of all families in the world. For example, 79% of the population in US reports difficult initiating, maintaining sleep and waking up freshly, in which falling into sleep and difficult waking up are most prevalent problems in general population. Additionally the sleeplessness sufferers reported unsatisfied performance, emotional problems and social difficulties more often and severe than normal sleepers. Therefore sleep enhancement is of great interests in both technical and market aspects.

Statistics indicate that 57% of sleeplessness sufferers in US have difficulty falling asleep a few nights per week. Though the short-term usage of sleeping pills may be useful for acute and situational sleeplessness, long-term use is not recommended because of the high potential risk of tolerance and dependency. Other safe and long-term effective alternatives are therefore needed.

It has been observed that people tend to easily fall asleep in a running vehicle such as car and train etc. The mimic motion of the vehicle can help sleep initiation.

US6,682,495 describes a vibrating bed comprising a basic bed having a bed frame, casters secured on the basic bed, a lateral vibrating bed plate movably ridden on the casters, a mechanism for reciprocally moving the vibrating bed plate with respect to the bed frame, a connecting rod adjusting device and tension coil springs for dampening the vibrations of the vibrating bed plate as the vibrating bed plate reaches a predetermined horizontal, or lateral, position. The vibrating bed has a timer switch enabling the user to select a time period. Furthermore the user can select a frequency by adjusting a motor speed controller.

SUMMARY OF THE INVENTION

It is an object of the present invention to provide an improved system for moving a sleeping surface.

It is a further object of the present invention to provide a sleeping surface provided with such an improved system.

It is a still further object of the present invention to provide a method for moving a sleeping surface.

According to a first aspect of the invention there is provided a system for moving a sleeping surface for a human being, comprising

- at least one actuator for moving the sleeping surface,
- a movement controller for controlling the actuator,

characterized by
a physiological state estimation unit for estimating a physiological state of the human being on the basis of a sensed value of at least one sensed physiological parameter of said human being, wherein the operation of the movement controller is dependent on the estimated physiological state.

According to a second aspect of the invention there is provided a sleeping surface provided with such a system.

According to a third aspect of the invention there is provided a method for moving a sleeping surface for a human being, comprising

- providing at least one actuator for moving the sleeping surface,
- controlling the at least one actuator,

characterized by
estimating a physiological state of the human being on the basis of a sensed value of at least one sensed physiological parameter of said human being, wherein the actuator is controlled dependent on the estimated physiological state.

By estimating the physiological state and taking this estimated physiological state into account when controlling the actuator, sleep can be more efficiently induced and maintained. For example once it is detected that the human being is asleep the intensity of the movements of the actuator may be reduced, for example by reducing a movement frequency or amplitude. While movements mimicking the motion of a car may induce sleep, they may prevent the human being from attaining deep sleep. Accordingly by the measures of the present invention a better relaxation of the human being is achieved. This results in the additional advantage of a relatively modest overall power consumption as unnecessary motion by the actuator is prevented.

The physiological parameters may be estimated in a contact-free or in a contact-based manner. Here term contact-free is used for systems wherein the physiological parameters of a subject are established without physical contact with the subject, whereas the term contact-based is used for systems wherein the physiological parameters of a human are established using physical contact with the subject.

Examples of contact-free methods of establishing such physiological parameters are the use of a microphone to detect the breathing pattern or the use of inductive measurement of bio-impedance as disclosed

in International Application WO2006/129212. The above examples clearly do not form an exhaustive list of all possible methods of contact-free physiological parameter estimation. A distinct advantage of contact-free estimation over contact-based estimation is that it is less intrusive as it does not require the user to attach sensors to the body.

A further advantage of the use of contact-free parameter estimation is that the sensors may be integrated in the sleeping surface or in the system for moving the sleeping surface.

Alternatively, a contact-based estimation may be used, e.g. by means of a wearable ECG measurement system as disclosed in International Application WO2006/111875. Likewise a wrist mounted heart monitoring device may be used as described in WO2007/072239, this device is due to its form factor preferable to WO2006/111875 and may communicate the physiological parameters to the movement controller by means of a wireless transmission. The advantage of contact based estimation being that the reliability of the established parameters generally is higher at the cost of minor discomfort to the user.

BRIEF DESCRIPTION OF THE DRAWINGS

These and other aspects are described in more detail with reference to the drawing. Therein:

> Fig. 1 shows a sleeping surface provided with a system for moving the sleeping surface,
> Fig. 2 shows a physiological state estimation unit in an embodiment of the system,
> Fig. 3 shows a movement control unit and various peripheral units,
> Fig. 4 shows in more detail the mechanical construction of an embodiment of the sleeping surface and the actuators,
> Fig. 4A shows a detail of this mechanical construction,
> Fig. 5 schematically illustrates operation of an embodiment of a system according to the invention,
> Fig. 6A and 6B respectively show a first and a second exemplary movement pattern,
> Fig 7 shows a further aspect of a movement pattern,
> Fig. 8 shows a learning algorithm for settings of an operational mode of the system,
> Fig. 9 shows a learning algorithm for settings of an operational mode of the system.

DETAILED DESCRIPTION OF EMBODIMENTS

In the following detailed description numerous specific details are set forth in order to provide a thorough understanding of the present invention. However, it will be understood by one skilled in the art that the present invention may be practiced without these specific details. In other instances, well known methods, procedures, and components have not been described in detail so as not to obscure aspects of the present invention.

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity. Embodiments of the invention are described herein with reference to cross-section illustrations that are schematic illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing. Thus, the regions illustrated in the figures are schematic in nature and their shapes are not intended to illustrate the actual shape of a region of a device and are not intended to limit the scope of the invention.

It will be understood that when an element or layer is referred to as being "on", "connected to" or "coupled to" another element or layer, it can be directly on, connected or coupled to the other element or layer or intervening elements or layers may be present. In contrast, when an element is referred to as being "directly on," "directly connected to" or "directly coupled to" another element or layer, there are no intervening elements or layers present. Like numbers refer to like elements throughout. As used herein, the term "and/or" includes any and all combinations of one or more of the associated listed items.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section. Thus, a first element, component, region, layer or section discussed below could be termed a second element, component, region, layer or section without departing from the teachings of the present invention.

Spatially relative terms, such as "beneath", "below", "lower", "above", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the exemplary term "below" can encompass both an orientation of above and below. The device may be otherwise oriented (rotated 90 degrees or at other orientations) and the spatially relative descriptors used herein interpreted accordingly.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In case of conflict, the present specification, including definitions, will control. In addition, the materials, methods, and examples are illustrative only and not intended to be limiting.

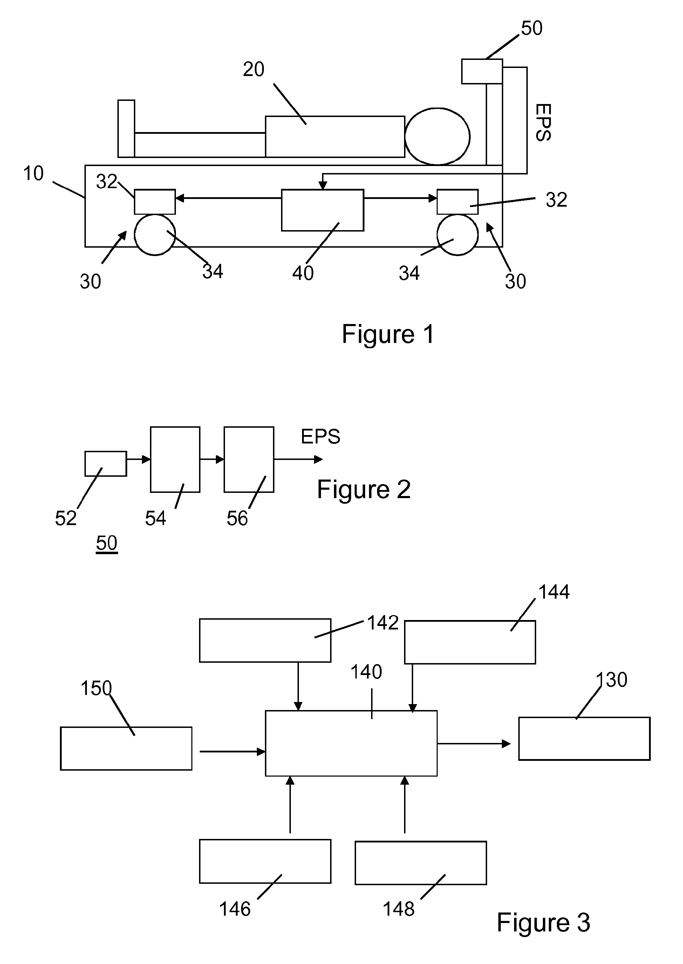

Figure 1 schematically shows a system for moving a sleeping surface 10 for a human being 20. The sleeping surface, a bed 10, is arranged at rotatable balls 34 that are moved by a motor 32. The combination of rotatable ball 34 and the motor 32 forms an actuator 30 for moving the sleeping surface. The system further comprises a movement controller 40 for controlling the actuator 30.

The system further comprises a physiological state estimation unit 50 for estimating a physiological state of the human being on the basis of a sensed value of at least one sensed physiological parameter of said human being 20. The operation of the movement controller is dependent on the estimated physiological state EPS. To that end the movement controller 40 has an input coupled via signal line EPS to the physiological state estimation unit 50.

Figure 2 shows an embodiment of the physiological state estimation unit 50. The physiological state estimation unit 50 comprises a sensor 52 for sensing a physiological parameter. In one embodiment the sensor 52 is a microphone that senses the breathing of the human being. In another embodiment the sensor 52 is a heart signal detector, and in still another embodiment the sensor 52 is arranged to sense muscle activity or brain activity. The signal from the sensor 52 is amplified and converted into a digital signal by an amplification and conversion circuit 54. The amplification and conversion circuit 54 provides a digital signal to a state recognition circuit 56. The state recognition circuit 56 estimates a physiological state of the human 20 on the basis of this digital signal. The state recognition circuit 56 may for example determine that the physiological state is "awake" if the breathing rate of the human being 20 is greater than a predetermined value, and determine that the physiological state is "asleep" if the breathing rate of the human being 20 is less than that predetermined value. The predetermined value may be set by the user or by a learning algorithm. Likewise the state recognition circuit 56 may for example determine that the physiological state is "awake" if the heart beat rate of the human being 20 is greater than another predetermined value, and determine that the physiological state is "asleep" if the heart beat rate of the human being 20 is less than the another predetermined value. The predetermined value may be set by the user or by a learning algorithm. Alternatively, methods are known to determine the physiological state from EEG measurements. Also position and movement are indicators for the physiological state of the human being. These can be observed by a camera. The observed image can be corrected for motion caused by the actuators, and after motion correction a physiological state of the human being can be detected by pattern recognition techniques.

Various measurements may be combined to obtain a more reliable physiological state estimation. Instead of merely detecting a binary state "asleep" or "awake", alternatively a more detailed state estimation may be given. For example a more detailed state estimation may indicate the state as "stressed", "relaxed but awake", "REM-sleep", "light-sleep" and "deep-sleep".

Figure 3 shows a part of a further embodiment of a system according to the invention. Parts therein corresponding to those in Figure 1 have a reference number that is 100 higher.

In the embodiment shown, the movement controller 140 is coupled to a user interface module (UI module) 142. The UI-module 142 has input facilities, such as a keyboard, touch screen, a microphone, a camera that allows the user to set the system according to his own preferences. The preferences are stored in a memory 144. The UI-module 142 may further comprise output facilities, e.g. a screen, a speaker, control lamps that may inform the user about current settings of the system. The movement controller 140 further is coupled to a duration timer 146 and an alert timer 148. The duration timer 146 may serve to enforce the maintenance of various operational modes of the system during a predetermined time-interval. The alert timer 148 may be set to provide an "awake" signal at a predetermined point in time. Both the duration timer 146 and the alert timer 148 may be set by the user via the UI-module 142.

The sleeping surface and the at least one actuator may for example be constructed as described in US6,682,495

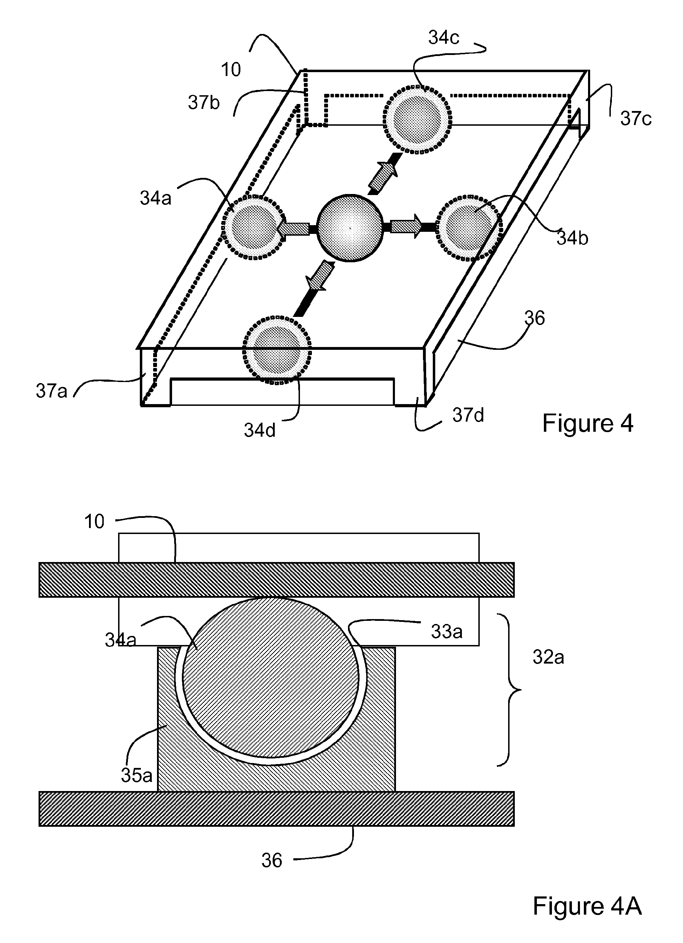

However, Figure 4 shows a more preferred embodiment that is relatively noise free. As shown therein, the sleeping surface 10 is arranged at one or more balls 34a, ..., 34d, that are rotatably housed in a socket mounted at a bottom plate 36. By way of example this is shown in more detail in Figure 4A for one of the balls 34a. Therein the ball 34a is arranged with play 33a in the socket 35a. The socket 35a may form a ball-bearing for the ball 34a, but alternatively may be an oil bearing. An actuator 32a may be formed by the combination of the socket 35a and the ball 34a. For example the ball 34a may comprise permanent magnetic elements, while the socket 35a comprises coils that are actuated by the movement controller 40. The weight of the sleeping surface 10 is supported by the balls 34a, ..., 34d, and by the supporting columns 37a,.., 37d that are arranged shiftable on the bottom plate 36.

In other embodiments, actuators may be used that provide for a linear movement of the sleeping surface 10.

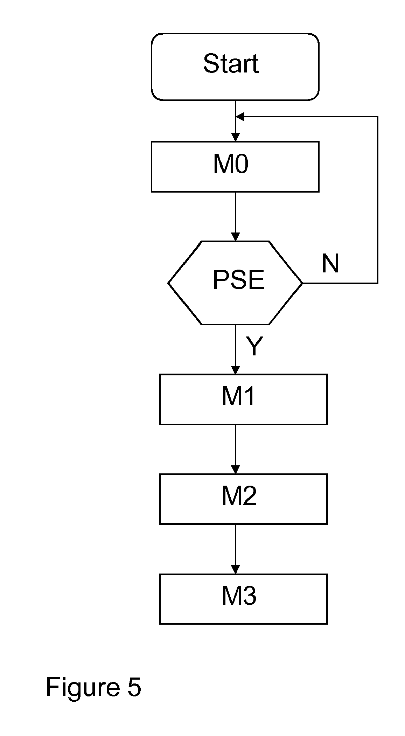

Figure 5 schematically illustrates operation of an embodiment of a system according to the invention.

After startup of the system (Start), the system assumes an initial operational mode M0, wherein the one or more actuators 30 are controlled to move the sleeping surface according to a default movement pattern, or according to a preferred movement pattern stored by the user in the memory 144.

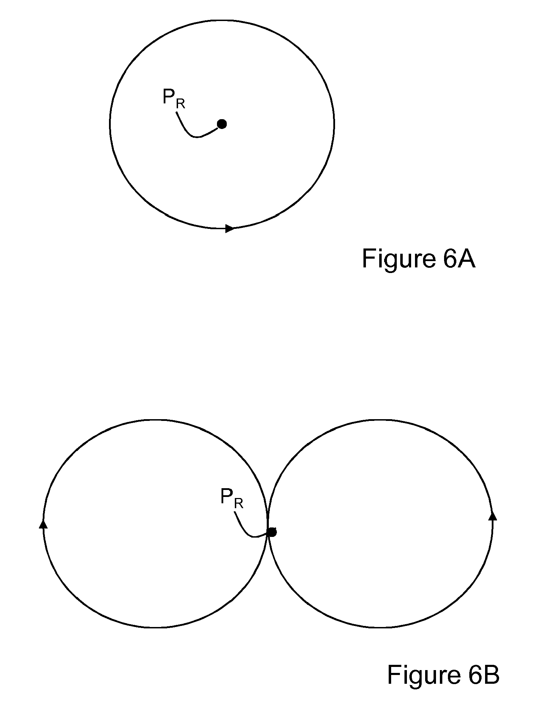

Examples of typical movement patterns are shown in Figures 6A, 6B. According to the pattern shown in Figure 6A the actuators 30 move the sleeping surface in a horizontal plane with a movement centered according to a reference point $P_R$.

In this case the movement is a circular movement around the reference point $P_R$.

Figure 6B shows another example of a movement pattern. Therein the sleeping surface moves in an 8-like pattern that is centered relative to reference point $P_R$.

Centered movements in a horizontal plane are considered the most comfortable, while uncentered or vertical movements may in some cases cause dizziness and/or disorientation.

Note that the movement pattern is a translation of the sleeping surface. The orientation of the sleeping surface 10 remains substantially constant.

The movement typically has a repetition frequency in the order of 2 Hz, e.g. from 1 to 5 Hz. A substantially lower frequency e.g. less than 0.1 Hz would have no measurable effect. A frequency higher than 5 Hz would not result in a better sleep induction than is already obtained with lower frequencies and would merely result in unnecessary power consumption. A substantially higher frequency e.g. 10 Hz would be uncomfortable and prevent the user from sleeping.

Dependent on the repetition frequency f, the amplitude A, corresponding to the radius is typically in a range from a few mm, e.g. 2 mm up to a few cm, e.g. 5 cm. An amplitude significantly smaller than 2mm, e.g. 1 mm would not have a sleep inducing effect. An amplitude significantly larger than 5 cm, e.g. 10 cm would be uncomfortable, and merely result in a high power consumption.

The jerk $j_x$, i.e. the third derivative of the position is both related to the frequency and the amplitude according to

$$j_x = -8\pi^3 f^3 A \cos 2\pi f t$$

A similar relation exists for the jerk in orthogonal direction $j_y$. For most individuals the jerk occurring during the initial operational mode and the first and the second main operational mode should be limited to a maximum of 5 m.s$^{-3}$, although for some individuals a higher jerk level, e.g. 10 m.s$^{-3}$ may be allowable. As a result preferably a system according to the present invention is a system wherein the jerk is less than 10 m/s$^3$ or alternatively less than 5 m/s$^3$ when it should be designed to accommodate more selective users.

Based on the maximum of 5 m.s$^{-3}$, a suitable upper limit $A_{max}$ for the amplitude during those operational modes is

$$A_{\max} \approx 0.02 f^{-3}$$

Accordingly for a frequency f = 2 Hz the value for $A_{max}$ is 2.5 mm, for a frequency of f = 1 Hz the value for $A_{max}$ is 20 mm, and for a frequency of 0.5 Hz the value for $A_{max}$ is 160 mm.

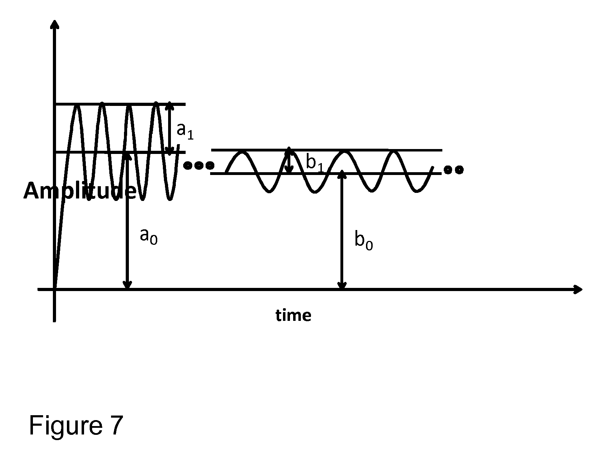

It may be advantageous that the amplitude of the movements is variable instead of constant. This is shown in Figure 7 by way of example. In this example, during the initial operational mode of the system, the amplitude varies with a deviation $a_1$ around a nominal value $a_0$. And in the first main operational mode the amplitude varies with a deviation $b_1$ around a nominal value $b_0$.

The amplitude is varied with a frequency that is small as compared to the frequency of the movement of the at least one actuator. For example if the actuator is moved with 2 Hz, then the amplitude is varied with a frequency of 0.5 Hz or lower, e.g. 0.2 Hz. Varying the amplitude has the advantage that mode changes, e.g. from the initial operational mode to the first main operational mode are cloaked. This reduces a risk that the user notices these changes and awakes.

A preferred setting may be defined by the user.

While in the initial operational mode M0 the state of the user 20 is estimated (PSE). State estimation may take place continuously, but may alternatively take place periodically, for example every minute or every five minutes. As long as it is detected that the user is still awake, indicated by case N, the system maintains its operational mode M0.

Once it is estimated that the user is asleep, or a period after that, e.g. after 5 or 15 minutes, the movement controller 40 assumes a first main operational mode M1, wherein the at least one actuator 30 is controlled to move with a gradually reducing amplitude or frequency. The gradual reduction of the amplitude or frequency may take place in a course of e.g. 1 or 2 hours. Subsequently the movement controller assumes a second main operational mode M2, wherein the at least one actuator 30 is controlled to move with an intermittent motion. The intermittent motion may for example have a duty cycle of 5-20%, e.g. 10%, e.g. motion may occur each 10 minutes and have a duration of 1 minute, or occur each hour and have a duration of about 5 minutes.

At a predetermined point in time the movement controller 40 may enter awake-up mode M3, wherein the movement controller 40 controls the at least one actuator 30 to move according to a movement pattern that results in an awakening of the human being.

The movement pattern applied during the wake-up mode M3, may correspond to that during the other operational modes M0- M2, but the amplitude and/or frequency preferably is higher than during the initial operating mode M0.

Instead of determining settings of the movement controller beforehand, or leaving this up to the user, it is alternatively possible that the movement controller is adaptive, i.e. determines itself what are the most suitable settings for the movement pattern.

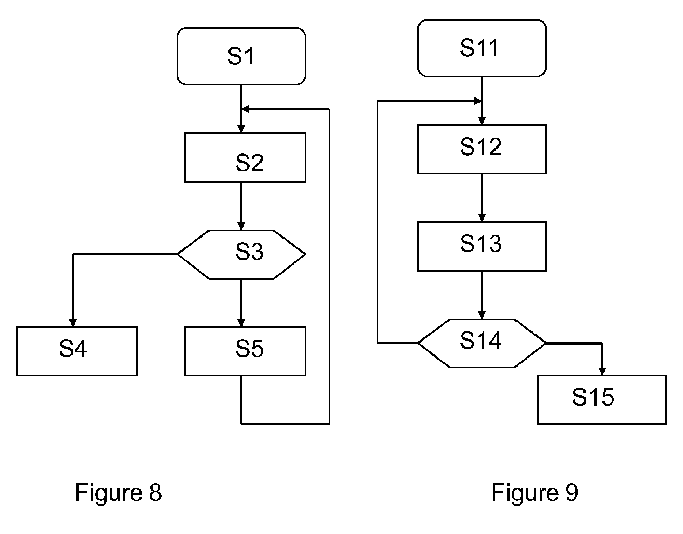

Figure 8 shows an example of a learning algorithm for learning settings of mode M0. In step S1, the moment that the user goes to bed, the movement controller starts with an initial setting for amplitude and frequency. Physiological parameters are measured in step S2, and subsequently the physiological state of the user is determined in step S3. If it is determined in step S3 that the user tends to sleep this confirms that the initial settings are suitable to induce sleep with that user, and these settings are stored in step S4. If rather it is determined in step S3 that the user tends to wake-up, different settings are generated in step S5, and control flow returns to step S2. The sequence of steps S2, S3, S5 is repeated until suitable settings are found for the purpose of inducing sleep.

Figure 9 shows an example of a learning algorithm for learning settings of mode M3. In step S11, a moment t1 is determined that is a period of time τ2 before the scheduled wake-up time. Then, in step S 12 the movement controller initiates a movement with an initial setting for amplitude and frequency. Physiological parameters are measured in step S13, and subsequently the physiological state of the user is determined in step S14. If it is determined in step S 14 that the user tends to wake up, this confirms that the initial settings are suitable for wake-up purposes with that user, and these settings are stored in step S 15. If rather it is determined in step S 14 that the user tends to stay asleep, control flow returns to step S12, where different settings are generated. The sequence of steps S12, S 13, S 14 is repeated until suitable settings are found for waking up purposes.

A system according to the present invention may be incorporated in a single device; this is particularly the case wherein contact-free sensors are employed. As a result the entire system can be integrated in a single device, preferably is a floor-mounted bed. As will be clear to the skilled person two systems according to the present invention may readily be combined into a larger bed so as to accommodate a sleep inducing facility for two individuals.

It is noted that due to the fact that the sleep surfaces need to be able to move in order to facilitate sleep induction, sufficient clearance should be provided between the respective sleep surfaces so as to enable the mutual sleep surfaces to move in the same as well as opposing directions.

Moreover when two systems are used side by side, the sensor means may need to be adapted so as to isolate the relevant physiological signals for the respective individuals. For example in case microphones are use, two directional microphones may be used. Alternatively two omni-directional microphones located at opposing sides of the bed may be used to emulate directional microphones by means of signal processing on the registered audio input signals. It should be noted that the inputs from both microphones can be used in the process, e.g. by cancelling out audible signals from one individual when estimating the physiological state of the other individual.

It will be clear to the skilled person combining two systems that certain components may be shared, such as the movement controller. The movement controller may be implemented so as to provide two actuator outputs, but to use one and the same movement controller to control the actuators for both sleeping surfaces. In this case the processor could perform all processing in a time-sequential or parallel manner.

It will be understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. In the claims the word "comprising" does not exclude other elements or steps, and the indefinite article "a" or "an" does not exclude a plurality. A single component or other unit may fulfill the functions of several items recited in the claims. Signal processing functions may be implemented by dedicated hardware, by a suitably programmed general purpose processor or a by a combination of both. The mere fact that certain measures are recited in mutually different claims does not indicate that a combination of these measures cannot be used to advantage.

Any reference signs in the claims should not be construed as limiting the scope.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

## Claims

1. System for moving a sleeping surface (10) for a human being (20), comprising

   - at least one actuator (30) for moving the sleeping surface,
   - a movement controller (40) for controlling the actuator,

   **characterized by**
   a physiological state estimation unit (50) for estimating a physiological state of the human being on the basis of a sensed value of at least one sensed physiological parameter of said human being, wherein the operation of the movement controller is dependent on the estimated physiological state.

2. System according to claim 1, wherein said at least one sensed physiological parameter is sensed with a contact-free sensor.

3. System according to claim 1, wherein the movement controller (40) has a first main operational mode (M1), wherein the at least one actuator (30) is controlled to move with a gradually reducing amplitude.

4. System according to claim 3, wherein said first main operational mode (M1) is initiated upon detection that the estimate physiological state is a sleeping state.

5. System according to claim 3 or 4, wherein the movement controller (40) has a second main operational mode (M2) following the first main operational mode (M1), wherein the at least one actuator (30) is controlled to move with periodic motion that is slower than a periodic motion during the first main operational mode

6. System according to claim 3 or 4, wherein the movement controller (40) has a second main operational mode (M2) following the first main operational mode (M1), wherein the at least one actuator (30) is controlled to move with an intermittent motion.

7. System according to claim 3, wherein the movement controller (40) has an initial operational mode (M0), wherein the at least one actuator (30) is controlled to move according to a default movement pattern

8. System according to claim 1, further comprising an alert-timer (148) for detecting a user settable point in time, wherein the alert-timer is arranged to initiate a wake-up mode (M2) of the movement controller (40), wherein the movement controller controls the at least one actuator (30) to move according to a movement pattern that results in an awakening of the human being.

9. System according to claim 1, wherein the at least one actuator (30) is arranged to move the sleeping surface (10) in a horizontal plane with a movement centered according to a reference point ($P_R$).

10. System according to claim 1, wherein the jerk is less than 10 m/s$^3$.

11. System according to claim 1, wherein the at least one sensed physiological parameter of said human being is at least one of:

    - a breathing rate,
    - a heart beat rate,
    - a muscle activity and
    - a brain activity.

12. System according to claim 1, wherein the movement controller is provided with a learning algorithm for learning suitable settings for an operational mode of the movement controller.

13. Sleeping surface (10) provided with a system for moving the sleeping surface according to one of the previous claims.

14. Sleeping surface (10) according to claim 13, wherein the at least one actuator (32a) comprises a ball (34a) that is rotatably housed in a socket (35a) mounted at a bottom plate (36), and that supports the sleeping surface.

15. Method for moving a sleeping surface for a human being, comprising

    - providing at least one actuator for moving the sleeping surface,
    - controlling the at least one actuator,

    **characterized by**
    estimating a physiological state of the human being on the basis of a sensed value of at least one sensed physiological parameter of said human being, wherein the actuator is controlled dependent on the estimated physiological state.

Figure 1

Figure 2

Figure 3

34c

10

37b

37c

34a

34b

36

37a

34d

37d

Figure 4

10

34a

33a

32a

35a

36

Figure 4A

Figure 5

P<sub>R</sub>

Figure 6A

P<sub>R</sub>

Figure 6B

Figure 7

Figure 8

Figure 9

Europäisches
Patentamt

European
Patent Office

Office européen
des brevets

**EUROPEAN SEARCH REPORT**

Application Number

EP 08 17 2261

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 198 11 207 C1 (KOPP BURKHARD [DE]; MALBERG HAGEN [DE]) 5 January 2000 (2000-01-05) * the whole document * | 1-15 | INV. A61M21/00 A47C21/00 A61G7/00 A61H1/00 |
| X | WO 98/39996 A (NIMS INC [US]) 17 September 1998 (1998-09-17) * the whole document * | 1-15 | |
| X | US 6 155 976 A (SACKNER MARVIN A [US] ET AL) 5 December 2000 (2000-12-05) * the whole document * | 1-15 | |
| X | US 2007/179334 A1 (GROVES LAURIE L [US] ET AL) 2 August 2007 (2007-08-02) * the whole document * | 1-15 | |
| X | DE 25 52 488 A1 (HEINRICH JOACHIM) 13 October 1977 (1977-10-13) * the whole document * | 1-15 | |
| X | US 4 619 270 A (MARGOLIS FREDERICK J [US] ET AL) 28 October 1986 (1986-10-28) * the whole document * | 1,15 | TECHNICAL FIELDS SEARCHED (IPC) A61M A47C A61G A61H |

The present search report has been drawn up for all claims

| Place of search | Date of completion of the search | Examiner |
|---|---|---|
| Munich | 6 May 2009 | Borowski, Aleksander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 08 17 2261

This annex lists the patent family members relating to the patent documents cited in the above-mentioned European search report.
The members are as contained in the European Patent Office EDP file on
The European Patent Office is in no way liable for these particulars which are merely given for the purpose of information.

06-05-2009

| Patent document cited in search report | | | Publication date | Patent family member(s) | | Publication date |
|---|---|---|---|---|---|---|
| DE 19811207 | C1 | | 05-01-2000 | NONE | | |
| WO 9839996 | A | | 17-09-1998 | AT | 331457 T | 15-07-2006 |
| | | | | DE | 69835092 T2 | 25-01-2007 |
| | | | | EP | 1006845 A1 | 14-06-2000 |
| | | | | JP | 2002515804 T | 28-05-2002 |
| US 6155976 | A | | 05-12-2000 | NONE | | |
| US 2007179334 | A1 | | 02-08-2007 | NONE | | |
| DE 2552488 | A1 | | 13-10-1977 | NONE | | |
| US 4619270 | A | | 28-10-1986 | NONE | | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6682495 B
- WO 2006129212 A
- WO 2006111875 A
- WO 2007072239 A